# EUROPEAN PATENT SPECIFICATION

(11) **EP 3 542 845 B1**
(45) Date of publication and mention of the grant of the patent: **22.12.2021**
(21) Application number: 16921852.6
(22) Date of filing: 21.11.2016
(51) Int. Cl.: A61M 16/04, A61M 25/02

(54) **A DEVICE FOR HOLDING ENDOTRACHEAL TUBES**
VORRICHTUNG ZUM HALTEN VON ENDOTRACHEALTUBEN
DISPOSITIF DE FIXATION DE TUBES ENDOTRACHÉAUX

(43) Date of publication of application: 25.09.2019
(73) Proprietor: Clip Fab R&D Medical Instruments, 08017 Barcelona (ES)
(72) Inventor: FERRANDIZ CATALAN, Antonio, 08017 Barcelona (ES); CASANOVAS RODRIGUEZ, Jose Miguel, 08017 Barcelona (ES)
(74) Representative: Isern Patentes y Marcas S.L.
(86) International application number: PCT/ES2016/070829
(87) International publication number: WO 2018/091753

(56) References cited:
- WO-A1-01/91838
- WO-A1-99/65553
- WO-A1-2010/033109
- WO-A1-2016/116916
- CN-U- 203 090 158
- US-A- 5 402 776
- US-A- 5 806 516
- US-A1- 2002 095 118
- US-A1- 2009 084 377
- US-A1- 2013 087 152
- US-A1- 2014 261 441
- US-A1- 2014 261 463
- US-A1- 2016 235 935
- US-B2- 9 233 221

## Description

### Object of the invention.

This invention relates to a device for holding endotracheal tubes, intended to secure at least one endotracheal tube with respect to a patient's mouth and comprising: a bite tube that defines a passage zone for at least one endotracheal tube; a front plate and an adjustable retainer, preferably inflatable, joined to the bite tube and forming means to limit displacement of the bite tube in an axial direction towards the interior and exterior of the mouth of the patient, respectively, and in a transversal direction; and means for immobilising at least one endotracheal tube with respect to the bite tube in an axial and transversal direction.

This holding device presents characteristics aimed at facilitating its fitting over the endotracheal tube or tubes, both in an axial and a transversal direction.

### Scope of the invention.

This invention is relevant to hospitals, mobile healthcare units, emergency care and medical or veterinary care in general.

### Prior art.

Currently, catheters and endotracheal tubes are widely used in the medical health field for different purposes such as to provide oxygen or liquids to the body or eliminate secretions from inside the body.

Once the patient has been intubated correctly, the endotracheal tube needs to be held to prevent it from moving forwards inadvertently into or out of the patient.

Traditionally, the endotracheal tube is held in place with respect to the patient using straps encircling said tube, outside the patient's mouth and fixed behind the patient's head or neck.

As well as being awkward, slow and insecure, this holding method is impractical as it prevents or at least significantly obstructs other operations such as cleaning or treating the patient's mouth; as well as impairing the appearance of the intubated patient.

Another disadvantage is that the endotracheal tube, which passes through the interior of the patient's mouth, can be bitten by the patient themselves causing its obstruction and the consequent loss of functionality of the endotracheal tube.

These problems were solved in part by the device described in document US 20020095118 A1 (WO2001091838 A1) which comprises: a tubular-shaped bite block with a distal end and a proximal end; an inflatable balloon attached to the distal end of said bite block and a shield extending from the proximal end of said bite block.

This tubular-shaped bite block is intended to be introduced into the patient's mouth 20 and to define a passage closed at the perimeter for the introduction of the endotracheal tube; said bite block acts as a protection of the endotracheal tube preventing the patient from being able to bite or obstruct it.

The inflatable balloon located on the distal end of the bite block has a dual function - on the one hand, it is positioned inside the patient's mouth behind their teeth so preventing the displacement of the device towards the outside of the mouth; and, on the other, it exerts pressure on the endotracheal tube that passes through the interior of the bite block, preventing the axial displacement of the endotracheal tube **30** with respect to the holding device.

This prior art also provides for the device to be able to incorporate two inflatable balloons to perform the two previous functions separately; in both cases, the inflatable balloon or balloons have one inflation line from the exterior of the patient's mouth.

This device presents specific disadvantages for use in terms of the ease of its fitting and removal.

Given that the block or bite tube has only one axial orifice for fitting it around the endotracheal tube or tubes, it has to be assembled in the axial direction by introducing one of the ends of said tubes via said axial orifice; furthermore, once the patient has been intubated, the endotracheal tube must be held in the correct position with one hand and the balloon or balloons that secure the device with respect to the patient's mouth in the direction of removal must be inflated, and the endotracheal tube secured with respect to the device, with the other hand.

There are other documents about the prior art related to the invention.

The document US9233221B2 discloses a bite block and methods of use thereof are disclosed. The bite block may be configured to encompass a tube such as an endotracheal tube, and to protect the mouth of an intubated patient from clenching damage.

The document US2014/261441A1 discloses a retention system for use with a bridle installed in a nose of a patient includes a member coupled to a medical device that is suspended from a nose of a patient and a connector for coupling to a bridle that is installed in a nose of a patient. The medical device extends into the mouth of a patient or is suspended adjacent the face of a patient.

The document US5402776A discloses an endotracheal tube holder that includes a face plate assembly which is attached to a patient's head by an adjustable headband. The face plate assembly is formed with a face plate adapted to be positioned over the mouth of the patient and is formed with an open end channel terminating with a V-shaped notch over which is superposed a tube holding block that is affixed to the outer surface of the face plate, and also includes a matching V-shaped notch in which an endotracheal tube can be fixedly positioned by a thumb screw, a bite block being integrally secured to the inner surface of the face plate which is also provided with an aligned V-shaped notch. The face plate also includes an opening for ready access to the patient's mouth, as might be required.

The document US2009/084377A1 discloses a endotracheal tube protector for use with an endotracheal tube comprises an elongated tubular member having a first end configured to extend out of a patient's mouth when in use and a second end configured to enter the throat of the patient. The tubular member has openings in the first and second ends and defines a substantially cylindrical cavity with an interior wall configured to hold an endotracheal tube therewithin.

The tubular member has a longitudinal slit for inducing radial expansion of the cavity and a pair of opposed bosses formed longitudinally in the tubular member at positions adjacent to the slit. The opposed bosses are configured to project into the cavity and to transmit a clamping force onto the endotracheal tube when contacted by the upper teeth of the patient when the endotracheal tube protector is in use and oriented such that the slit faces toward the roof of the patient's mouth.

The document WO99/65553A1 discloses an assembly for fixing a tube for medical purposes to a patient's mouth, the tube being fixed to the patient's head. According to the invention the assembly comprises a tube clamping means which can be attached to the tube in a detachable manner, which tube clamping means is provided with positioning means connected thereto, which position the tube clamping means during use with the help of flexible, detachable securing means that are to be arranged around the patient's head, the tube clamping means being designed such that it can be clamped around the tube after arranging the tube in the patient's mouth without disturbing the location of the tube.

The document US2014/261463A1 discloses a bite block for an endotracheal tube has a tubular wall with a pair of opposed ends, a central opening along a length of the bite block between the pair of opposed ends, an interior wall surface, and an accessory line channel positioned between the interior wall surface and an endotracheal tube extending through the central opening. The accessory line channel is formed by a wall segment that is recessed into the interior wall surface, the wall segment being thinner than a thickness of the tubular wall adjacent the wall segment. The bite block can be part of or attachable to an endotracheal tube holding device attachable to a patient.

The document WO2016/116916A1 discloses a securing apparatus, for securing an endotracheal tube to a patients' body, the securing apparatus comprising: at least one cylinder indentation, for surrounding the endotracheal tube, perimeter of the at least cylinder indentation being larger than that of the endotracheal tube, for allowing sliding the cylinder indentation in relation to the endotracheal tube; at least two parallel plates, extending from the at least one cylinder indentation, for confining teeth of the patient; and at least one embracing element, connected to the at least one cylinder indentation, for embracing the endotracheal tube, thereby the securing apparatus allows securing the endotracheal tube in relation to the patient's teeth, being advantaged of having a well defined reliable stationary location regarding the patient's body, and thereby allowing adjusting the location before said securing.

The document WO2010/033109A1 discloses a securement system secures an endotracheal tube or other medical article in position upon a patient and arrests movement of the endotracheal tube. The securement system includes a support member and a retainer. The retainer defines a channel configured to receive the endotracheal tube. A head securement member attaches the support member to the head of the patient. The securement system can include a soft gel for placement against the patient's skin and well as a track for securing the endotracheal tube at a plurality of locations relative to the support member. Advantageously, the securement system can maintain an endotracheal tube in position upon a patient and allow access to the patient's oral cavity.

The document US5806516A discloses an endotracheal tube stabilizer includes an elongate frame having a transverse tube channel with an opening sized to radially receive an endotracheal tube. Straps are provided for securing the frame to the head of a patient with the frame bridging and the tube adjacent to the patient's mouth. A clamp has a body and a distal foot. The clamp body is attached to the frame with the distal foot traveling in a arcuate path relative to the frame from an open position remote from the opening of the transverse tube channel to a select operative position blocking the opening of the tube channel. In this manner, with an endotracheal tube received in the channel, the distal foot, when in an operative position, clamps the endotracheal tube in fixed position relative to the frame within the slot.

Despite the existence of said prior art, there is a need to develop a device for securing endotracheal tubes with respect to a patient's mouth that simplifies the fitting and removal of the device with respect to the endotracheal tube or tubes, once the patient has been intubated.

### Description of the invention.

The invention is defined in independent claim 1. The dependent claims are disclosing preferred embodiments of the invention. The endotracheal tube holding device that is the object of this invention is intended to secure at least one endotracheal tube with respect to a patient's mouth and is of a type that comprises: a bite tube that defines a passage zone for at least one endotracheal tube; a front plate and an adjustable retainer, constituted by an inflatable balloon, joined to the bite tube and which form means for limiting displacement of the bite tube in an axial direction towards the interior and exterior of the mouth of the patient, respectively, and in a transversal direction; and means for immobilising at least one endotracheal tube with respect to the bite tube in an axial and transversal direction.

This device has special design features aimed at solving the problems set out above satisfactorily and facilitating its fitting and removal with respect to the endotracheal tube once the patient has been intubated, so that it can be removed easily, for example to treat or clean the patient's mouth, without the need to extubate the patient or disconnect the endotracheal tube from any external device, for example, for supplying liquid or oxygen to the patient.

To achieve the objectives proposed, this device has the special feature of a lateral opening along the entire length of the bite tube, the front plate and the adjustable retainer for lateral or transversal introduction of at least one endotracheal tube into the passage zone defined by the bite tube.

The front plate and adjustable retainer are arranged externally with respect to the bite tube so immobilising the device with respect to the patient's mouth only, as it is attached via the pressure exerted by the adjustable retainer when inflated, preferably on the internal part of the teeth, trapping the teeth between the adjustable retainer, preferably, and the front plate.

When in position for use, the front plate is adjusted between the patient's teeth and lips to prevent lip blisters or sores.

The means of immobilisation of the endotracheal tube comprise a pressure clamp attached to the front plate, open towards the lateral opening of the device and arranged in parallel to an exterior face of the front plate.

According to the invention, these characteristics allow the device to be assembled and disassembled in the transversal or lateral direction, on any intermediate point of the endotracheal tube, simply by introducing said tube via a lateral opening of the device, defined for this purpose on the bite tube, the adjustable retainer, the front plate and the pressure clamp.

This characteristic simplifies the fitting and removal of the device with respect to the endotracheal tube or tubes considerably, once the patient is intubated, as the ends of said tubes do not need to be free or accessible.

According to the invention, the pressure clamp that is placed parallel to an exterior face of the front plate comprises an end part attached to this front plate and two elastically flexible arms, which define toothed concave portions, facing each other, to secure at least one endotracheal tube in the passage zone of the bite tube.

This front plate comprises stops for securing the arms of the pressure clamp, elastically bent into an open position of this clamp; this enables the device to be fitted on any intermediate area of the endotracheal tube or tubes and moved in an axial direction with respect thereto, for example, during the correct positioning of the device with respect to the patient's mouth, maintaining the clamp in an open position.

For the pressure clamp to be in an operational position, its arms simply need to be released from the retaining stops so that they then exert pressure on the endotracheal tube or tubes passing through the interior of the device, preventing its relative displacement in an axial direction.

Therefore, said pressure clamp makes it possible to hold and release the endotracheal tube or tubes that pass through the interior of the device, rapidly and simply, without the need to use or manipulate external elements such as an inflation tube or pressure syringe operated manually.

The design of the holding clamp may vary according to its adaptation to different endotracheal tube sizes.

### Description of the figures.

To supplement this description and facilitate the understanding of the characteristics of the invention, this description is accompanied by a set of drawings in which the following have been represented with an illustrative rather than limitative character:
Figure 1 is a front perspective view of one embodiment of the device for holding endotracheal tubes according to the invention.
Figure 2 is a back elevation view of the holding device in the previous figure.

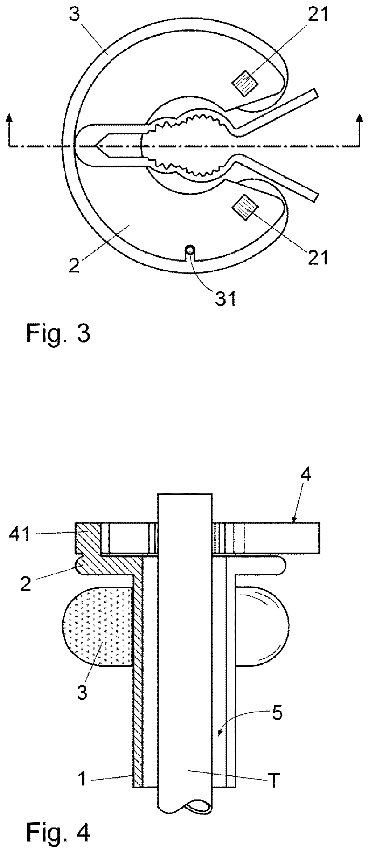

Figure 3 is a front elevation view of the holding device in the previous figures.
Figure 4 is a top plan view of the holding device in the previous figures divided by the horizontal plane shown in figure 3.
Figure 5 is a bottom plan view of the holding device in the previous figures with an adjustable retainer, constituted by an inflatable balloon, shown in a deflated position.
Figure 6 is a front elevation view of the holding device in the previous figures during the introduction of an endotracheal tube in the transversal direction, in its interior and with the pressure clamp held in an open position.

### Preferred embodiment of the invention.

In the embodiment example shown the attached figures, the device for holding the endotracheal tubes comprises: a bite tube (1); a front plate (2) and an adjustable retainer (3) arranged externally with respect to the bite tube (1) and substantially perpendicular to said bite tube (1); and a pressure clamp (4) attached to the front plate (2).

According to the invention, the adjustable retainer (3) is constituted by an inflatable balloon.

This inflatable balloon (3) is intended to be positioned and inflated inside the patient's mouth, trapping the device against the patient's teeth, preventing the device from moving in an axial direction towards the exterior of the mouth; whilst the front plate (2) is intended to be positioned between the patient's teeth and lips, preventing the axial displacement of the device towards the interior of the mouth.

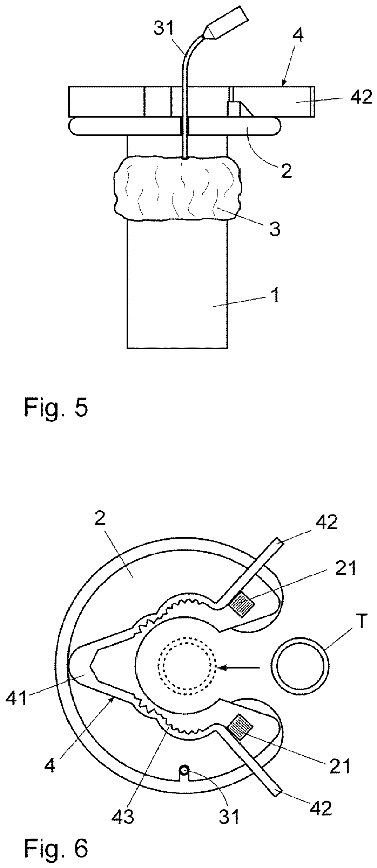

As can be observed in the attached figures, this device comprises a lateral opening (5) along the entire length of the bite tube (1), the front plate (2) and the inflatable balloon (3) for lateral or transversal introduction of at least one endotracheal tube (T) into the passage zone (11) defined by the bite tube (1), as shown in figure 6.

The inflatable balloon (3) has an inflation tube (31) for the connection of a pressure syringe that can be actuated manually or any other suitable device for supplying air or fluid under pressure and the inflation thereof.

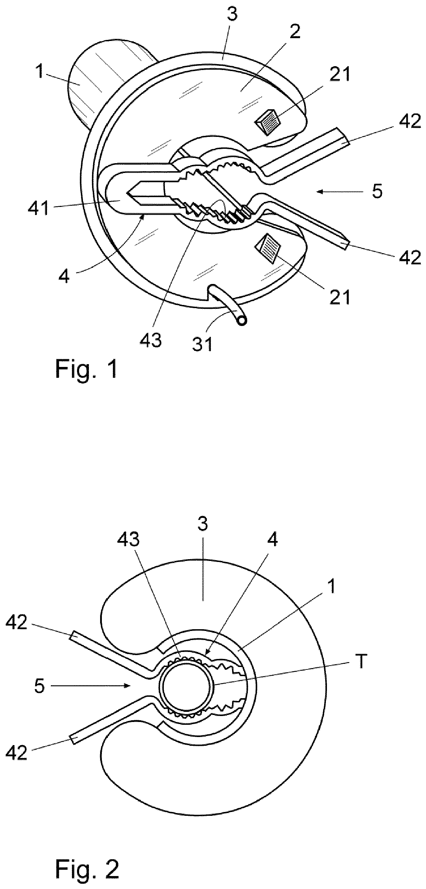

The pressure clamp (4) is arranged in parallel to an exterior face of the front plate (2) and comprises: an end part (41) attached to this front plate and two elastically flexible arms (42), which define toothed concave portions (43), facing each other, to secure at least one endotracheal tube (T) in the passage zone (12) of the bite tube (1) as shown in figure 2.

The front plate (2) comprises stops (21) for securing the arms (42) in the open position of the clamp (4) represented in figure 6.

These stops (21) have ramps oriented towards the lateral opening (5) of the device to facilitate the positioning of the arms (42) of the pressure clamp (4) in the open position, it being sufficient to separate the arms (42) in the direction of the opening of the clamp (4) until they pass said stops 21 and are held in the open position represented in figure 6 for this purpose.

To return the clamp (4) to the operational position represented in figure 2, the arms (42) simply need to be bent slightly in the front direction for them to be released from the stops (21) and return to said operational position as a result of the action of the elastic energy accumulated therein.

Once the nature of the invention has been described sufficiently, along with a preferred embodiment example, it is noted that the materials, shape, size and arrangement of the parts described may be altered for the appropriate purposes provided that this does not change the essential characteristics of the invention claimed below.

## Claims

1. Device for holding endotracheal tubes, intended to secure at least one endotracheal tube with respect to a patient's mouth and comprising: a bite tube (1)
that defines a passage zone (11) for at least one endotracheal tube (T), a front plate (2) and an adjustable retainer (3) joined to the bite tube (1) and which form means for limiting displacement of the bite tube (1) in the axial direction towards the interior and exterior of the mouth of the patient respectively; and means for immobilising at least one endotracheal tube with respect to the bite tube (1) in the axial direction;
wherein:
- the device comprises a lateral opening (5) along the entire length of the bite tube (1), the front plate (2) and the adjustable retainer (3) for lateral or transversal introduction of at least one endotracheal tube (T) into the passage zone (11) defined by the bite tube (1),
- the front plate (2) and the adjustable retainer (3) are arranged externally with respect to the bite tube (1), **characterised in that**
- the adjustable retainer (3) is constituted by an inflatable balloon.

2. Device as claimed in claim 1, **characterised in that** the means of immobilisation of the endotracheal tube comprise a pressure clamp (4) attached to the front plate (2) open towards the lateral opening (5) of the device and arranged in parallel to an exterior face of the front plate.

3. Device as claimed in claim 2, **characterised in that** the pressure clamp (4) comprises an end part (41) attached to the front plate (2) and two elastically flexible arms (42) with toothed concave portions (43), facing each other, to secure at least one endotracheal tube (T) in the passage zone (11) of the bite tube (1).

4. Device as claimed in claim 3; **characterised in that** the front plate (2) comprises stops (21) for securing the arms (42) elastically bent into an open position of the pressure clamp (4).

## Patentansprüche

1. Vorrichtung zum Halten von Endotrachealtuben, die dazu bestimmt ist, mindestens einen Endotrachealtubus in Bezug auf den Mund eines Patienten zu sichern und die Folgendes umfasst: einen Beißtubus (1),
der eine Durchgangszone (11) für mindestens einen Endotrachealtubus (T) definiert, eine Frontplatte (2) und eine verstellbare Halterung (3), die mit dem Beißtubus (1) zusammengefügt sind und Mittel zur Begrenzung der Verschiebung des Beißtubus (1) in axialer Richtung zum Inneren bzw. Äußeren des Mundes des Patienten bilden; und Mittel zum Immobilisieren mindestens eines Endotrachealtubus in Bezug auf den Beißtubus (1) in axialer Richtung;
wobei:
- die Vorrichtung eine laterale Öffnung (5) über die gesamte Länge des Beißtubus (1), der Frontplatte (2) und der verstellbaren Halterung (3) zum lateralen oder transversalen Einführen mindestens eines Endotrachealtubus (T) in die Durchgangszone (11), die durch das Beißtubus (1) definiert wird, umfasst,
- die Frontplatte (2) und die verstellbare Halterung (3) bezüglich des Beißtubus (1) außen angeordnet sind, **dadurch gekennzeichnet, dass**
- die verstellbare Halterung (3) durch einen aufblasbaren Ballon gebildet ist.

2. Vorrichtung nach Anspruch 1, **dadurch gekennzeichnet, dass** die Mittel zur Immobilisierung des Endotrachealtubus eine Druckklemme (4) umfassen, die an der Frontplatte (2) angebracht ist, die zur lateralen Öffnung (5) der Vorrichtung hin offen ist und parallel zu einer Außenfläche der Frontplatte angeordnet ist.

3. Vorrichtung nach Anspruch 2, **dadurch gekennzeichnet, dass** die Druckklemme (4) ein an der Frontplatte (2) angebrachtes Endteil (41) und zwei elastisch biegsame Arme (42) mit einander zugewandten gezahnten konkaven Abschnitten (43) umfasst, um mindestens einen Endotrachealtubus (T) in der Durchgangszone (11) des Beißtubus (1) zu sichern.

4. Vorrichtung nach Anspruch 3; **dadurch gekennzeichnet, dass** die Frontplatte (2) Anschläge (21) zum Sichern der in einer offenen Position der Druckklemme (4) elastisch gebogenen Arme (42) umfasst.

## Revendications

1. Dispositif de fixation de tubes endotrachéaux, destiné à retenir au moins un tube endotrachéal par rapport à la bouche d'un patient et comprenant : un tube de morsure (1)
qui définit une zone de passage (11) d'au moins un tube endotrachéal (T), une plaque frontale (2) et un élément de retenue ajustable (3) reliés au tube de morsure (1) et qui forment des moyens pour limiter le déplacement du tube de morsure (1) dans la direction axiale respectivement vers l'intérieur et l'extérieur de la bouche du patient ; et des moyens pour immobiliser au moins un tube endotrachéal par rapport au tube de morsure (1) dans la direction axiale ;
dans lequel :
- le dispositif comprend une ouverture latérale (5) sur toute la longueur du tube de morsure (1), de la plaque frontale (2) et de l'élément de retenue ajustable (3) pour l'introduction latérale ou transversale d'au moins un tube endotrachéal (T) dans la zone de passage (11) définie par le tube de morsure (1),
- la plaque frontale (2) et l'élément de retenue ajustable (3) sont agencés extérieurement par rapport au tube de morsure (1), **caractérisé en ce que**
- l'élément de retenue ajustable (3) est constitué d'un ballon gonflable.

2. Dispositif selon la revendication 1, **caractérisé en ce que** les moyens d'immobilisation du tube endotrachéal comprennent une pince de serrage (4) reliée à la plaque frontale (2) ouverte vers l'ouverture latérale (5) du dispositif et agencée parallèlement à une face extérieure de la plaque frontale.

3. Dispositif selon la revendication 2, **caractérisé en ce que** la pince de serrage (4) comprend une partie d'extrémité (41) reliée à la plaque frontale (2) et deux bras élastiquement flexibles (42) avec des parties concaves dentées (43), l'une en regard de l'autre, pour maintenir au moins un tube endotrachéal (T) dans la zone de passage (11) du tube de morsure (1).

4. Dispositif selon la revendication 3 ; **caractérisé en ce que** la plaque frontale (2) comprend des butées (21) pour maintenir les bras (42) pliés élastiquement dans une position ouverte de la pince de serrage (4).
